# FASCICULE DE BREVET EUROPEEN

(11) **EP 1 401 561 B1**
(45) Date de publication et mention de la délivrance du brevet: **03.05.2006**
(21) Numéro de dépôt: 02740857.4
(22) Date de dépôt: 04.06.2002
(51) Int. Cl.: B01F 17/00, B01F 17/56, C08B 37/00

(54) **NOUVELLE UTILISATION DE COMPLEXES D'INCLUSION DE CYCLODEXTRINE**
NEUE VERWENDUNG VON CYCLODEXTRINEINSCHLUSSKOMPLEXEN
NOVEL USE OF CYCLODEXTRIN INCLUSION COMPLEXES

(30) Priorité: 08.06.2001 FR 0107499
(43) Date de publication de la demande: 31.03.2004
(73) Titulaire: SOCIETE D'EXPLOITATION DE PRODUITS POUR LES INDUSTRIES CHIMIQUES ( S.E.P.P.I.C.), 75321 Paris Cedex 07 (FR)
(72) Inventeur: MILIUS, Alain, F-06000 Nice (FR); TROUVE, Gérard, F-81100 Castres (FR); BOITEUX, Jean-Pierre, F-81710 Saix (FR); BOJINOVA, Tzvetana, F-75321 Paris cedex 07 (FR); DE VIGUERIE, Nancy, F-31000 Toulouse (FR); POINSOT, Verena, F-31120 Lacroix Falgarde (FR); RICO LATTES, Isabelle, F-31650 Auzielle (FR)
(74) Mandataire: Conan, Philippe Claude
(86) Numéro de dépôt international: PCT/FR2002/001876
(87) Numéro de publication internationale: WO 2002/100524

(56) Documents cités:
- EP-A- 0 470 452
- EP-A- 0 773 229
- WO-A-94/01518
- WO-A-95/03709
- WO-A-96/20008
- WO-A-97/36972
- DE-A- 4 136 325
- FR-A- 2 550 445
- US-A- 3 565 887
- US-A- 4 438 106
- PATENT ABSTRACTS OF JAPAN vol. 007, no. 147 (C-173), 28 juin 1983 (1983-06-28) & JP 58 058139 A (NIHON SAAFUAKUTANTO KOGYO KK), 6 avril 1983 (1983-04-06) cité dans la demande
- DATABASE WPI Section Ch, Week 198320 Derwent Publications Ltd., London, GB; Class A96, AN 1983-47500K XP002193319 & JP 58 058139 A (NIPPON SURFACTANT KK), 6 avril 1983 (1983-04-06) cité dans la demande

## Description

La présente invention a pour objet l'utilisation en tant que tensioactifs de complexes d'inclusion cyclodextrine-corps gras, notamment pour la préparation d'émulsions.

Elle trouve notamment application dans les domaines cosmétiques, pharmaceutiques, alimentaires et industriels.

On sait que les tensioactifs non ioniques qui représentent une part importante (environ 40 % en 1998) de la production mondiale de tensioactifs sont, pour la quasi-totalité d'entre eux, obtenus par la mise en oeuvre de procédés de synthèse comportant une étape de condensation de l'oxyde d'éthylène. De ce fait, ces composés peuvent comporter des impuretés, comme par exemple le dioxanne ou l'oxyde d'éthylène, qui sont généralement considérées comme des produits toxiques et préjudiciables à la santé.

C'est pourquoi de nouveaux tensioactifs non-ioniques ont été développés, lesquels comportent une partie hydrophile dérivée de sucre ou de glycérol. Parmi ces composés, on peut citer les alkylpolyglucosides, les esters de sorbitan, les esters de méthyl glucose, les esters de sucrose ou esters de saccharose ou sucroesters, les amides d'acides aldoniques, les éthers de polyglycérol, les esters de polyglycérol, les polyglycérol méthyl glucose esters, les esters de lactitol, les esters de lactose, les esters de glucose, les éthers de glucose, les éthers de sucrose, les alkylglucamines, les amides de glycamines ou glycamides.

Cependant, bien qu'exempts d'impuretés provenant de la condensation de l'oxyde d'éthylène, ces composés sont tous obtenus par des réactions chimiques utilisant des catalyseurs, qui sont également susceptibles de générer des sous-produits non identifiés, éventuellement toxiques.

Dans ces conditions, il existe un besoin non satisfait à ce jour, de disposer de nouveaux tensioactifs non ioniques, parfaitement exempts d'impuretés, et susceptibles d'être obtenus de façon satisfaisante à l'échelle industrielle.

Il a été découvert, et ceci constitue le fondement de la présente invention, que certains composés obtenus par inclusion de corps gras dans la cavité d'une cyclodextrine, et dénommés "complexes d'inclusion", présentent des propriétés tensioactives remarquables qui permettent d'envisager leur utilisation comme agents émulsionnants ou bien encore comme agents moussants dans des applications variées.

Ces complexes d'inclusion, caractérisés par une liaison de type non-covalente entre la cyclodextrine hydrophile et le corps gras hydrophobe sont obtenus par la mise en oeuvre de réactions douces ne faisant intervenir aucun catalyseur chimique. Ils sont donc dépourvus de sous-produits toxiques.

En outre, par un choix judicieux des cyclodextrines et corps gras les constituant, il est possible de moduler à volonté les propriétés tensioactives, et en particulier la balance hydrophile/lipophile, de ces complexes d'inclusion et de disposer ainsi d'une nouvelle famille de composés couvrant l'ensemble des propriétés des tensioactifs : hydrotrope, solubilisant, mouillant, moussant, détergent et émulsionnant:

Il est à noter qu'il existe déjà des composés tensioactifs dérivés de cyclodextrines.

Cependant, ces composés connus, qui sont obtenus par estérification d'une ou plusieurs fonctions hydroxyles de la cyclodextrine par un acide gras, comportent une liaison hydrophile hydrophobe de type covalent issue d'une synthèse chimique traditionnelle avec catalyseur et solvant et présentent donc les mêmes inconvénients que les composés tensioactifs décrits précédemment.

En outre, les applications de ces composés sont peu nombreuses en raison de leur stabilité limitée à l'hydrolyse.

Il est encore à noter que la littérature décrit quelques exemples d'utilisation mettant à profit la formation de complexes d'inclusion entre des cyclodextrines et des corps gras.

C'est ainsi que la formation de complexes entre des cyclodextrines et des corps gras a notamment été mise à profit :
- pour extraire des composés indésirables comme le cholestérol (US 4,880,573) ou les acides gras libres (US 5,560,950) de différentes huiles alimentaires ;
- pour préparer ou stabiliser des émulsions à usage alimentaire comme des mayonnaises ou bien encore des émulsions industrielles comme les fluides de coupe (WO 94/01518).

Dans ce dernier cas, c'est l'aptitude des cyclodextrines à former des complexes amphiphiles par inclusion partielle des constituants de la phase grasse que l'on cherche à mettre à profit pour obtenir une émulsion.

Cependant, cette méthode est empirique et n'offre pas de garantie de succès puisqu'il est connu que les cyclodextrines peuvent conduire, dans des conditions d'utilisation proches, au résultat inverse, à savoir casser des émulsions stables en complexant les corps gras de ces émulsions (WO 95/34363).

Ces procédés connus conduisent donc au meilleur des cas à des émulsionnants à base de cyclodextrine :
- dont la structure est aléatoire puisque des phases grasses de différentes natures sont généralement utilisées dans une émulsion et qu'on ne peut prédire celle(s) de ces phases grasses qui sera (seront) effectivement incluse(s) dans la cyclodextrine ;
- dont la stoechiométrie est inconnue ; et
- dont la quantité réellement formée n'est pas connue puisque la préparation se fait in situ.

Il en résulte que ces procédés connus ne permettent pas de maîtriser les trois paramètres essentiels nécessaires pour sélectionner un émulsionnant approprié pour la préparation d'une émulsion stable, à savoir sa structure, sa balance hydrophile - lipophile (HLB) et sa concentration.

Dans le document JP58-58139, est décrit un procédé de préparation d'émulsions huile dans eau essentiellement caractérisé en ce qu'il comprend :
- la préparation d'une composition émulsionnante par dispersion d'une cyclodextrine dans du glycérol ou dans une solution aqueuse de glycérol, puis l'ajout d'un tensioactif soluble dans l'huile ;
- l'addition au mélange ainsi obtenu d'une huile, telle que l'huile de paraffine ou une huile de silicone, en formant ainsi une émulsion huile dans glycérol stable ; et
- l'addition d'eau à l'émulsion ainsi obtenue, en formant ainsi une émulsion huile dans eau.

Il est indiqué dans ce document antérieur que le trioléylphosphate, les alcools aliphatiques et les acides gras ayant de 8 à 20 atomes de carbone peuvent être utilisés en lieu et place du tensioactif soluble dans l'huile dans la première étape précitée.

Le procédé décrit dans ce document antérieur est plus satisfaisant que les procédés de l'état de la technique discutés précédemment, notamment en ce qu'il permet une meilleure maîtrise de la structure de l'émulsionnant.

Cependant, ce procédé présente encore de très nombreux inconvénients :
- il ne permet pas de connaître la quantité d'émulsionnant formé (on ne sait même pas s'il se forme) ni sa stoechiométrie et par conséquent sa balance HLB ;
- il est relativement compliqué en ce qu'il comporte la préparation intermédiaire d'une émulsion huile dans glycérol stable ;
- il impose l'utilisation de grandes quantités de glycérol ;
- il impose une fabrication limitée à la quantité nécessaire pour la fabrication de l'émulsion et n'est donc pas adapté à une utilisation à l'échelle industrielle.

Il a été découvert d'une façon tout à fait inattendue qu'il était possible de préparer des complexes à base de cyclodextrine et de corps gras présentant des propriétés tensioactives parfaitement maîtrisées, et se présentant sous une forme adaptée à leur utilisation comme tensioactif, notamment comme émulsionnant ou bien encore comme agent moussant ou mouillant dans des applications variées.

Ainsi, selon un premier aspect, la présente invention concerne l'utilisation en tant que tensioactifs de complexes d'inclusion sous forme de poudre ou en dispersion aqueuse entre une cyclodextrine et un corps gras,, caractérisée en ce que le rapport molaire entre la cyclodextrine et le corps gras dans le complexe précité est inférieur ou égal à 1, en ce que la cyclodextrine précitée est choisie parmi une β-cyclodextrine, une hydroxypropyl-β-cyclodextrine, ou une méthyl-β-cyclodextrine et en ce que le corps gras précité est un alcool gras choisi parmi les alcools linéaires ou ramifiés, saturés ou insaturés, d'origine naturelle ou d'origine synthétique, et dont le nombre d'atomes de carbone est compris entre 8 et 36.

La cyclodextrine constituant les complexes d'inclusion selon l'invention est choisie parmi :
- une β-cyclodextrine, comme par exemple le produit commercialisé sous la dénomination KLEPTOSE® par la Société ROQUETTE, CAVANAX W7 par la Société WACKER-CHEMIE GmbH ou encore C. CAVITRON 82900 par la Société CERESTAR ;
- une hydroxypropyl-β-cyclodextrine, comme par exemple le produit commercialisé sous la dénomination CAVASOL W7 HP par la Société WACKER-CHEMIE GmbH ;
- une méthyl-β-cyclodextrine, comme par exemple le produit commercialisé sous la marque CAVASOL W7M par la société WACKER-CHEMIE GmbH.

Les alcools gras susceptibles d'être utilisés dans le cadre de la présente invention, sont des alcools linéaires ou ramifiés, saturés ou insaturés, d'origine naturelle ou synthétique, comme par exemple les alcools provenant de matières végétales (coprah, palmiste, palme), animales (suif), ou les alcools de Guerbet, et dont le nombre d'atomes de carbone est compris entre 8 et 36.

Les tensioactifs conformes à l'invention pourront être préparés de manière connue en soi, et seront avantageusement utilisés après avoir été isolés, de préférence sous forme solide ou en dispersion aqueuse.

Ces composés pourront être préparés à partir d'une solution ou d'une suspension de cyclodextrine et de corps gras.

Le procédé de préparation des complexes en solution consiste essentiellement :
- à mélanger intimement en solution au moins une cyclodextrine et un corps gras, à une température et pendant une durée suffisante pour obtenir une solution homogène ;
- à provoquer la précipitation du complexe d'inclusion, par refroidissement contrôlé de la solution homogène ainsi obtenue.

Dans ce procédé, l'eau sera généralement utilisée comme solvant unique, mais il peut être nécessaire d'y ajouter une faible quantité de solvant organique pour dissoudre le corps gras et améliorer sa solubilisation dans la phase aqueuse.

D'une façon générale, le mélange réactionnel constitué de la cyclodextrine, du corps gras et du solvant précité sera chauffé puis soumis à vive agitation pour conduire à une solution homogène.

Les conditions nécessaires à l'obtention d'une solution homogène (température du mélange réactionnel, intensité et durée de l'agitation) pourront être facilement déterminées par l'homme de métier.

Avantageusement, on travaillera à une température comprise entre 40 et 80°C, de préférence entre 50 et 70°C, sous vive agitation pendant une durée comprise entre 10 et 30 heures.

Dans ce procédé, la complexation (c'est-à-dire l'inclusion du corps gras dans la cyclodextrine) est réalisée de préférence sous pression normale par un refroidissement contrôlé de la solution homogène préalablement préparée, comprenant une première phase au cours de laquelle la température est progressivement abaissée, par exemple à une valeur de l'ordre de 4°C, et une deuxième phase au cours de laquelle le mélange réactionnel est maintenu à cette température basse, pendant une durée suffisante pour que le complexe cristallise, par exemple de l'ordre de 10 à 30 heures.

Le complexe ainsi obtenu pourra être isolé par exemple par filtration et éventuellement séché, broyé, tamisé, granulé pour son utilisation ultérieure en tant que tensioactif, sous forme solide.

Le procédé de préparation en solution est relativement long et peut nécessiter l'utilisation d'un solvant organique.

C'est pourquoi les tensioactifs utilisés dans le cadre de la présente invention seront de préférence préparés par un procédé en suspension plus rapide et qui évite le recours à un solvant organique. Celui-ci consiste pour l'essentiel :
- à additionner le corps gras à une suspension ou dispersion aqueuse de cyclodextrine, et
- à chauffer la suspension ou dispersion ainsi obtenue à une température de l'ordre de 30 à 70°C, de préférence de 40 à 60°C, sous agitation, pendant une durée comprise entre 1 et 8 heures, de préférence entre 1 et 3 heures.

La nature du corps gras et de la cyclodextrine sont susceptibles dans ce cas d'influencer la viscosité de la suspension, et de ce fait, les concentrations de cyclodextrine et de corps gras seront ajustées pour permettre l'obtention d'un mélange homogène entre la cyclodextrine et le corps gras.

Les conditions optimales d'obtention d'un complexe d'inclusion par le procédé en suspension pourront être facilement déterminées par l'homme de métier.

Le complexe obtenu pourra être isolé par exemple par séchage ou lyophilisation et éventuellement séché, broyé, tamisé, granulé pour son utilisation ultérieure en tant que tensioactif, sous forme solide.

Quel que soit le procédé de préparation choisi, le rapport molaire entre la cyclodextrine et le corps gras dans le mélange réactionnel permettant l'obtention des complexes d'inclusion formant les tensioactifs conformes à la présente invention, sera inférieur à 1.

Dans ces conditions, il a été observé, d'une façon tout à fait inattendue, que les complexes d'inclusion obtenus ont des propriétés tensioactives excellentes et présentent de ce fait un grand intérêt pour la préparation de compositions notamment mouillantes, moussantes, détergentes et émulsionnantes.

D'une façon tout à fait inattendue, il a été découvert que les complexes d'inclusion formés avec les alcools gras présentent des propriétés tensioactives et une stabilité nettement supérieures à celles des complexes d'inclusion formés avec d'autres corps gras de même longueur de chaîne hydrocarbonée, comme par exemple les acides gras ou les esters méthyliques d'acides gras.

En outre, d'une façon tout à fait surprenante, on a montré que les propriétés tensioactives des complexes de cyclodextrine et d'alcools gras pouvaient être significativement améliorées en utilisant pour leur préparation un excès stoechiométrique d'alcool gras.

Il a été constaté que les complexes de cyclodextrine et d'alcools gras, ayant 16 atomes de carbone ou plus, présentent des propriétés émulsionnantes remarquables permettant leur utilisation pour la préparation d'émulsions, notamment dans les domaines cosmétiques, pharmaceutiques, agricoles ou alimentaires.

D'une façon générale, ces complexes seront utilisés comme émulsionnant, en une quantité comprise entre 1 et 25% en poids, rapportée au poids total de l'émulsion.

Les émulsions préparées à partir de ces complexes comprendront en outre de préférence :
- de 5 à 75% en poids d'une phase aqueuse ; et
- de 20 à 90 % en poids, de préférence de 20 à 60% en poids, d'une phase grasse ou huileuse.

Cette phase grasse ou huileuse peut être constituée par une ou plusieurs huiles choisies parmi les huiles d'origine végétale, les huiles végétales modifiées, les huiles d'origine naturelle, les huiles minérales, les huiles synthétiques ou bien encore les corps gras d'origine végétale, animale ou synthétique.

Ces émulsions pourront également contenir, d'une façon optionnelle jusqu'à 10% en poids d'un co-émulsionnant, et jusqu'à 10% en poids d'un agent stabilisant.

Dans leur application en tant que tensioactifs émulsionnants, les complexes seront utilisés selon l'invention sous forme solide ou sous forme de dispersion aqueuse.

L'invention sera illustrée plus en détail par les exemples qui suivent, donnés uniquement à titre illustratif.

Dans ces exemples :
- les pourcentages sont exprimés en poids et la température est la température ambiante, sans indication contraire.
- les analyses en RMN (solvant : DMSO-d₆) ont été réalisées sur des spectromètres AC 200 BRUCKER à 400 MHz pour la RMN ¹H et à 75 MHz ou 100 MHz pour la RMN ¹³C ;
- les analyses en Spectrométrie de Masse (SM) ont été réalisées sur un spectromètre AUTOSPEC MICROMASS (Angleterre) en mode d'ionisation LSIMS positif, FAB au Cs à 16 kV, matrice : glycérol / thioglycérol 1 : 1 ;
- les analyses en Infra-Rouge (IR) ont été réalisées sur un spectrophotomètre IRTF PERKIN ELMER 1760 X avec séparatrice en KBr ;
- les analyses de calorimétrie différentielle (DSC) ont été réalisées sous atmosphère inerte (N₂), sur un appareil PYRIS 1 DSC PERKIN ELMER ;
- les mesures de tension de surface ont été réalisées sur un tensiomètre TD1 LAUDA associé à un bain thermostaté et sur un tensiomètre à gouttes KRUSS GmbH modèle DSA 10-Mk2 ;
- les points de fusion ont été mesurés par calorimétrie différentielle.

### Exemple 1 : Préparation du complexe d'inclusion entre la β-cyclodextrine (β-CD) et l'acide dodécanoïque (C₁₁H₂₃COOH) (exemple comparatif)

### A. Par un procédé en solution

1,416 g de β-CD commercialisée par la société Roquette sous l'appellation β-cyclodextrine Kleptose® sont solubilisés, à température ambiante, dans 76,6 mL d'eau distillée grâce à un apport d'énergie par ultrasons, durant 15 minutes. La solution obtenue est placée sous agitation magnétique, dégazée par un courant d'argon puis chauffée à 50°C. A cette solution on ajoute 7,7 mL d'une solution comportant 250 mg d'acide dodécanoïque dans l'acétone (cette dernière est obtenue après un léger chauffage). L'agitation est maintenue soit à 50°C, soit à une température allant jusqu'à 70°C, jusqu'à obtention d'une solution homogène (durée moyenne : 2 jours).

Le mélange réactionnel ainsi préparé est refroidi de 70°C à 4°C en 6 heures, puis maintenu à 4°C pendant 2 jours (temps nécessaire à la cristallisation et à la décantation du complexe). La phase surnageante est éliminée, puis le solide recueilli est séché (sur CaCl₂) dans une étuve à vide à température ambiante.

On obtient ainsi 1,204 g de produit solide blanc qui est ensuite caractérisé par RMN ¹H et ¹³C, SM-FAB, DSC, CCM, IR et point de fusion.

### B. Par un procédé en suspension

Une masse de 2,000 g d'acide dodécanoïque est broyée dans un mortier avec 12,823 g de β-cyclodextrine. Un volume de 20,0 mL d'eau distillée est ajouté pour obtenir une suspension qui est ensuite soumise à une agitation mécanique (300 trs/min) et est chauffée à 50°C pendant 5 heures. Puis, le mélange réactionnel est laissé refroidir lentement jusqu'à température ambiante (pendant 20 heures) et est séché par lyophilisation. Le produit est récupéré sous forme de poudre blanche et ensuite caractérisé par les techniques précitées.

### Exemple 2 : Préparation du complexe d'inclusion entre la β-cyclodextrine (β-CD) et le dodécanol (C₁₂H₂₅OH) (Selon l'invention)

### A. Par un procédé en solution

On opère selon le procédé décrit dans l'exemple 1 en utilisant 1,523 g de β-CD dissous dans 82,3 mL d'eau distillée et 250 mg de dodécanol dans 8,2 mL d'acétone.

On obtient ainsi 0,952 g de produit sous forme d'un solide blanc qui est analysé par les techniques citées dans l'exemple 1.

Dans ce cas, l'analyse par Spectrométrie de Masse est réalisée en utilisant NaI à 1 mg/mL comme agent d'ionisation. Cette analyse a permis de mettre en évidence un complexe de stoechiométrie 1:1.

| | |
|---|---|
| MH⁺ [β-CD/C₁₂H₂₅OH] | m/z = 1321 |
| MNa⁺ [β-CD/C₁₂H₂₅OH] | m/z = 1343 |

La mise en évidence du complexe par DSC (Differential Scanning Calorimetry) est basée sur la disparition du pic de fusion de la molécule « invitée ». Lorsqu'elle est complexée, celle-ci n'a pas la structure cristalline d'une molécule invitée libre lui permettant d'adsorber de l'énergie. Le point de fusion de la chaîne grasse disparaît lorsque tout l'alcool est complexé. Trois pics sont présents sur le spectre. Le premier correspond au dodécanol non complexé, tandis que les deux autres sont attribués à une déshydratation soit de la β-cyclodextrine non complexée, soit du complexe. Un pic caractéristique du complexe n'est pas attribué.

Le pic le plus intéressant est le premier : son aire étant proportionnel à la quantité du dodécanol libre permet de quantifier la complexation.

La relation (ΔH_{MI libre}/ΔH_{MI pure})* 100 = % _{MI non complexée} est utilisée pour calculer le pourcentage de la molécule invitée restant non complexée.

| | | |
|---|---|---|
| β-cyclodextrine (β-CD) | T = 157,5°C | ΔH = 350 ± 40 J.g⁻¹ |
| Dodécanol (C₁₂H₂₅OH) | T_{f} = 26,2°C | ΔH = 203 J/g |
| Complexe (β-CD / C₁₂H₂₅OH) | T_{f} = 24,4°C | ΔH =8,2 J.g⁻¹ |
| | T_{g} =138,4°C | ΔCₚ = 1,5 J.g⁻¹.°C⁻¹ |
| | T = 157,9°C | ΔH = 8,7 J.g⁻¹ |

Ainsi, la quantité exprimée en pourcentage massique de dodécanol non complexé est de 4 ± 0,8 %. Le rendement de complexation est donc de 50 %.

### B. Par un procédé en suspension

Une suspension de 13,922 g de β-cyclodextrine dans 20 mL d'eau distillée est préparée. Cette suspension est soumise à une agitation mécanique (300 trs/min) pendant 15 minutes et est chauffée à 50°C. A cette suspension sont ajoutés 2,005 g de dodécanol. L'agitation est maintenue pendant 1 heure supplémentaire et à une température moyenne de 50°C. Ensuite, il est refroidi lentement jusqu'à température ambiante, puis séché par lyophilisation. 14,096 g de poudre blanche sont récupérés. Le rendement de la complexation est de 95 %.

### Exemple 3 : Préparation du complexe d'inclusion entre la β-cyclodextrine (β-CD) et l'octadécanol (C₁₈H₃₇OH) par un procédé en solution (exemple selon l'invention)

Une solution de 1,05 g de β-CD dans 56,7 mL d'eau distillée est obtenue après apport d'énergie par ultrasons pendant 10 minutes à température ambiante. La solution obtenue est placée sous agitation magnétique, chauffée à 60°C et dégazée par un courant d'argon. A cette solution sont additionnés 250 mg d'octadécanol en solution dans 5,7 mL d'acétone (cette dernière est obtenue après un léger chauffage). L'agitation est maintenue 2 jours à cette température, jusqu'à disparition de la phase solide surnageante (chaîne grasse) laquelle disparition a en outre nécessité une addition supplémentaire de 2 mL d'acétone.

Le mélange réactionnel ainsi obtenu est refroidi lentement de 60 à 4°C puis maintenu à cette température durant 4 jours (temps nécessaire à la cristallisation et à la décantation). La phase surnageante est éliminée et la phase solide récupérée est séchée sous vide à température ambiante.

On obtient ainsi 0,542 g d'un solide blanc qui est ensuite caractérisé par les méthodes citées dans l'exemple 1.

### Exemple 4 : Préparation du complexe d'inclusion entre l'hydroxypropyl-β-cyclodextrine (HP-β-CD) et l'acide octadécanoïque (C₁₇H₃₅COOH) par un procédé en solution (exemple comparatif):

1,85 g de HP-β-CD commercialisée par la société Roquette sous l'appellation de LAB 1456 sont mis en solution, à température ambiante, dans 3,1 mL d'eau distillée. Une solution de 250 mg d'acide octadécanoïque dans 2 mL d'éthanol absolu est additionnée. Le mélange réactionnel est placé sous agitation magnétique, dégazé sous un courant d'argon et chauffé à 60°C pendant 2 jours.

On laisse ensuite refroidir lentement le mélange, qui prend l'aspect d'un gel, jusqu'à 4°C et on maintient cette température pendant 3 jours. Le gel est séché sous vide à température ambiante pendant 8 heures.

On obtient ainsi 2,088 g de produit, sous forme d'un solide blanc, qui est analysé par les méthodes citées dans l'exemple 1.

### Exemple 5 : Préparation du complexe d'inclusion entre l'hydroxypropyl-β-cyclodextrine (HP-β-CD) et le dodécanol (C₁₂H₂₅OH) par un procédé en solution (exemple selon l'invention)

On opère selon le procédé décrit dans l'exemple 6, en utilisant 2,83 g de HP-β-CD dans 4,7 mL d'eau distillée et 250 mg de dodécanol dans 4,0 mL d'éthanol absolu.

On obtient ainsi 2,426 g d'un solide blanc qui est analysé en utilisant les méthodes mentionnées dans l'exemple 1.

La mise en évidence du complexe a été réalisée par DSC. Quelques pics sont présents sur le spectre entre 50 et 120°C. L'HP-β-CD pure se déshydrate à 157°C et se dégrade au-delà de 300°C. D'une part, l'absence du pic correspondant à la fusion du dodécan-1-ol et, d'autre part, le déplacement vers les plus hautes températures du T_{g} de l'HP-β-CD prouvent la formation quantitative du complexe. En effet, une augmentation du T_{g} traduit une rigidification du composé qui, dans le cas présent, est due à une inclusion de la molécule invitée dans la cavité de la cyclodextrine.

| | | |
|---|---|---|
| Hydroxypropyl-β-cyclodextrine | | |
| (HP-β-CD) | T_{g} = 99,0°C | ΔCₚ = 7,9 g⁻¹.°C⁻¹ |
| Dodécanol (C₁₂H₂₅OH) | T_{f} = 26,2°C | ΔH = 203 J.g⁻¹ |
| Complexe (HP-β-CD.C₁₂H₂₅OH) | T_{f} = 55°C | ΔH = 2,2 J.g⁻¹ |
| | T_{f} = 59,8°C | ΔH = 3,7 J.g⁻¹ |
| | T_{g} = 116,4°C | ΔCₚ = 4,0 J.g⁻¹.°C⁻¹ |

### Exemple 6 : Préparation du complexe d'inclusion entre la méthyl-β-cyclodextrine (Me-β-CD) et le dodécanol (C₁₂H₂₅OH) par un procédé en solution (exemple selon l'invention)

7,030 g de méthyl-β-CD commercialisée par la Société WACKER-CHEMIE sous l'appellation CAVASOL® W7 M sont solubilisés à température ambiante dans 5 mL d'eau distillée grâce à un rapport d'énergie par ultrasons, durant 20 minutes. La solution obtenue est placée sous agitation magnétique, dégazée par un courant d'argon puis chauffée à 70°C. A cette solution est ajouté 1 g de dodécanol en solution dans 2,0 mL d'éthanol absolu. Le mélange réactionnel obtenu est maintenu à 70°C jusqu'à obtention d'une solution limpide (3 jours). Il est ensuite refroidi jusqu'à température ambiante pendant 3 jours, puis maintenu à +4°C (température à laquelle le mélange réactionnel prend l'aspect d'un gel) pendant 15 jours. Le gel obtenu, séché sous vide à température ambiante pendant 8 heures, permet d'obtenir le complexe sous forme d'une poudre blanche qui est analysée par les méthodes citées dans l'exemple 1.

### Exemple 7 : Préparation du complexe d'inclusion entre la β-cyclodextrine (β-CD) et l'hexadécanol (C₁₆H₃₃OH) par un procédé en solution (exemple selon l'invention)

Ce complexe est préparé dans les conditions de l'exemple 3 en substituant l'octadécanol par l'hexadécanol.

### Exemple 8 : Préparation du complexe d'inclusion entre la β-cyclodextrine (β-CD) et le dodécanoate de méthyle par un procédé en suspension (exemple comparatif)

Une suspension de 12,200 g de β-cyclodextrine dans 17,4 mL d'eau distillée est préparée puis soumise à une agitation mécanique (300 trs/min) pendant 15 minutes à une température de 50°C. A cette suspension sont ajoutés 2,016 g de dodécanoate de méthyle. L'agitation est maintenue pendant 3 heures supplémentaires à une température moyenne de 55°C. Ensuite, le mélange réactionnel est refroidi lentement jusqu'à température ambiante, puis séché par lyophilisation. 12,385 g de produit sous forme de poudre blanche sont récupérés. La caractérisation est faite par RMN ¹H dans D₂O.

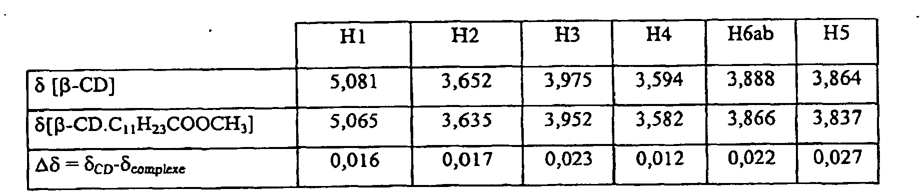

Les variations des déplacements chimiques pour tous les protons de l'hôte, mais surtout pour H3 et H5 (situés à l'intérieur de la cavité de la cyclodextrine) laissent supposer la formation du complexe. De plus, la présence des pics correspondants à la molécule invitée, qui est initialement insoluble dans l'eau prouve aussi que cette molécule est complexée.

### Mise en évidence de l'effet tensioactif par mesure des tensions de surface de solutions aqueuses des complexes des exemples 1, 2, 4, 5, 6 et 7

| | Complexe d'inclusion | Concentration mol.L⁻¹ | Tension de surface mN.m⁻¹ à 35°C |
|---|---|---|---|
| Exemple 1 | [β-CD.C₁₁H₂₃COOH] | 2,7.10⁻³ | 42,4 |
| Exemple 2 | [β-CD.C₁₂H₂₅OH] | 2,0.10⁻³ | 30,3 |
| Exemple 4 | [HP-β-CD.C₁₇H₃₅COOH] | 1,4.10⁻³ | 56,1 |
| Exemple 5 | [HP-β-CD.C₁₂H₂₅OH] | 1,4.10⁻³ | 55,2 |
| Exemple 6 | [Me-β-CD.C₁₂H₂₅OH] | 2,5.10⁻³ | 29,7* |
| Exemple 7 | [β-CD.C₁₆H₃₃OH] | 1,9.10⁻³ | 46,1 |

| | | | |
|---|---|---|---|
| * La tension de surface de ce complexe est mesurée à 25°C. | | | |

Les complexes d'inclusions sont tous des tensioactifs puissants puisqu'ils abaissent la tension de surface de l'eau pure de 72.0 mN.m⁻¹ à des valeurs comprises entre 29,7 et 56,1 mN.m⁻¹.

### Mise en évidence de la supériorité des complexes obtenus avec les alcools gras

### A. Propriétés tensioactives

Les propriétés tensioactives sont déterminées en mesurant la tension superficielle de solutions aqueuses de complexes obtenus à partir d'alcools gras et de complexes obtenus à partir d'acides gras ou d'ester d'acide gras de même longueur de chaîne.

| | Corps gras | Cyclodextrine | Stoechiométrie cyclodextrine / corps gras | Concentration mol.L⁻¹ | Tension superficielle à 20°C mN.m⁻¹ |
|---|---|---|---|---|---|
| Exemple 2 | Dodécanol | β-CD | 1/1 | 1.5 10⁻³ | 34,9 |
| Exemple 6 | Dodécanol | Me β-CD | 1/1 | 1.5 10⁻³ | 34,4 |
| Exemple 5 | Dodécanol | HP-β-CD | 1/1 | 1.5 10⁻³ | 42,8 |
| Exemple 1 | Acide dodécanoique | β-CD | 1/1 | 2.0 10⁻³ | 51,8 |
| Exemple 8 | Dodécanoate de méthyle | β-CD | 1/1 | 1.5 10⁻³ | 55,3 |

### B - Stabilité

La stabilité des complexes est déterminée en mesurant la tension superficielle de solutions aqueuses de complexes après 10 jours de stockage à 20°C :

| | Corps gras | Cyclodextrine | Concentration mol.L-1 | Tension superficielle à 20°C mN.m⁻¹ | Tension superficielle à 20°C mN.m⁻¹ après 10 jours |
|---|---|---|---|---|---|
| Exemple2 | Dodécanol | β-CD | 1.5 10⁻³ | 34,9 | 39,0 |
| Exemple 6 | Dodécanol | Me β-CD | 1.5 10⁻³ | 34,4 | 38,0 |
| Exemple 5 | Dodécanol | HP-β-CD | 1.5 10⁻³ | 42,8 | 56,3 |
| Exemple 1 | Acide dodécanoique | β-CD | 2.0 10⁻³ | 51,8 | 61,6 |

Il est clair à la lecture des tableaux qui précèdent que :
- les complexes d'alcools gras sont beaucoup plus tensioactifs que les complexes d'acides gras ou d'ester d'acide gras ;
- les complexes obtenus à partir d'alcools gras sont beaucoup plus stables que ceux obtenus à partir d'acides gras et les complexes obtenus avec β-CD et Me-β-CD sont plus stables que celui obtenu avec HP-β-CD.

### Mise en évidence de l'effet du rapport stoechiométrique cyclodextrine / alcools gras sur les propriétés tensioactives

Il a été découvert d'une façon tout à fait surprenante qu'en utilisant un excès stoechiométrique d'alcools gras pour la préparation des complexes, les propriétés tensioactives étaient significativement améliorées.

Cet aspect de l'invention est mis en évidence par l'exemple suivant :

### Exemple 9 : Préparation du complexe d'inclusion entre la β-cyclodextrine (β-CD) et le dodécanol dans un rapport stoechiométrique de 1/2

Une suspension de 13,939 g de β-cyclodextrine dans 20,0 mL d'eau distillée est préparée. Cette suspension est soumise à une agitation mécanique (300 trs/min) pendant 15 minutes et est chauffée à 50 °C à l'aide d'un bain de sable. A cette suspension sont ajoutés 4,005 g de dodécan-1-ol. Le mélange réactionnel est maintenu sous agitation pendant 1 heure supplémentaire à une température moyenne de 50 °C. Ensuite, il est refroidi lentement jusqu'à température ambiante, puis séché par lyophilisation. On obtient une poudre blanche qui est caractérisée par RMN ¹H et DSC. La quantité de dodécan-1-ol non complexé est de 0 %.

Les propriétés tensioactives sont déterminées en mesurant la tension superficielle de solution aqueuse de complexes.

| | Corps gras | Cyclodextrine | Stoechiométrie cyclodextrine / corps gras | Concentration mol.L⁻¹ | Tension superficielle à 20°C mN.m⁻¹ |
|---|---|---|---|---|---|
| Exemple2 | Dodécanol | β-CD | 1/1 | 1.5 10⁻³ | 34,9 |
| Exemple 9 | Dodécanol | β-CD | 1/2 | 1.5 10⁻³ | 24,5 |

Il est clair que les complexes obtenus à partir d'alcools gras sont d'autant plus tensioactifs que l'alcool gras est en excès stoeehiométrique.

## Revendications

1. Utilisation en tant que tensioactifs de complexes d'inclusion sous forme de poudre ou en dispersion aqueuse entre une cyclodextrine et un corps gras,, **caractérisée en ce que** le rapport molaire entre la cyclodextrine et le corps gras dans le complexe précité est inférieur ou égal à 1,**en ce que** la cyclodextrine précitée est choisie parmi une β-cyclodextrine, une hydroxypropyl-β-cyclodextrine, ou une méthyl-β-cyclodextrine, et **en ce que** le corps gras précité est un alcool gras choisi parmi les alcools linéaires ou ramifiés, saturés ou insaturés, d'origine naturelle ou d'origine synthétique, et dont le nombre d'atomes de carbone est compris entre 8 et 36.

2. Utilisation selon la revendication 1, **caractérisé en ce que** le rapport molaire entre la cyclodextrine et l'alcool gras est inférieur à 0,75.

3. Utilisation selon l'une quelconque des revendications 1 et 2, **caractérisée en ce que** le complexe précité est obtenu par la mise en oeuvre d'un procédé consistant essentiellement :
- à mélanger intimement en solution au moins une cyclodextrine et un corps gras, dans un rapport molaire inférieur ou égal à 1, à une température et pendant une durée suffisante pour obtenir une solution homogène ;
- à provoquer la précipitation d'un complexe d'inclusion, par refroidissement contrôlé de la solution homogène ainsi obtenue.

4. Utilisation selon l'une quelconque des revendications 1 et 2, **caractérisée en ce que** le complexe précité est obtenu par la mise en oeuvre d'un procédé consistant essentiellement :
- à additionner le corps gras à une suspension ou dispersion aqueuse de cyclodextrine ; et
- à chauffer la suspension ou dispersion ainsi obtenue à une température de l'ordre de 30 à 70°C, de préférence de 40 à 60°C, sous agitation, pendant une durée comprise entre 1 et 8 heures, de préférence entre 1 et 3 heures.

5. Utilisation selon la revendication 3 ou 4, **caractérisé en ce que** le complexe d'inclusion précité est isolé, par exemple par filtration, par séchage ou lyophilisation et éventuellement séché, broyé, tamisé, granulé pour son utilisation ultérieure sous forme solide.

6. Procédé de préparation d'une émulsion, **caractérisé en ce qu'**il comprend la préparation d'un complexe d'inclusion cyclodextrine/corps gras par la mise en oeuvre d'un procédé selon la revendication 3 ou 4, sa dispersion dans de l'eau puis l'ajout d'une phase grasse sous agitation.

7. Procédé de préparation d'une émulsion, **caractérisé en ce qu'**il comprend la préparation d'un complexe d'inclusion cyclodextrine/corps gras par la mise en oeuvre d'un procédé selon la revendication 3 ou 4, sa dispersion dans une phase grasse puis l'ajout d'eau sous agitation.

8. Emulsions, **caractérisée en ce qu'**elles sont obtenues par la mise en oeuvre du procédé selon l'une des revendications 6 ou 7 et **en ce qu'**elles comprennent :
- de 1 à 25% en poids d'au moins un complexe d'inclusion tel que défini à l'une quelconque des revendications 1 ou 2 ou obtenu par la mise en oeuvre du procédé selon l'une quelconque des revendications 3 ou 4;
- de 5 à 75% en poids d'eau ; et
- de 20 à 90% en poids d'une phase grasse ou huileuse.

## Patentansprüche

1. Verwendung von Einschlußkomplexen zwischen einem Cyclodextrin und einem Fettkörper, in Pulverform oder in wäßriger Dispersion als Tenside, **dadurch gekennzeichnet, daß** das Molverhältnis zwischen dem Cyclodextrin und dem Fettkörper in dem genannten Komplex 1 oder weniger als 1 beträgt, das das genannte Cyclodextrin aus der Gruppe β-Cyclodextrin, Hydroxypropyl-β-Cyclodextrin bzw. Methyl-β-Cyclodextrin stammt und daß der genannte Fettkörper ein Fettalkohol aus der Gruppe der geradkettigen oder verzweigten, gesättigten oder ungesättigten Alkohole natürlichen oder synthetischen Ursprungs stammt und eine Kohlenstoffatomanzahl zwischen 8 und 36 aufweist.

2. Verwendung nach Anspruch 1, **dadurch gekennzeichnet, daß** das Molverhältnis zwischen dem Cyclodextrin und dem Fettalkohol weniger als 0,75 beträgt.

3. Verwendung nach einem der Ansprüche 1 und 2, **dadurch gekennzeichnet, daß** der genannte Komplex mit Hilfe eines Verfahrens erhalten wird, das im wesentlichen aus den folgenden Schritten besteht:
- inniges Mischen von mindestens einem Cyclodextrin und einem Fettkörper in Lösung in einem Molverhältnis von 1 oder weniger als 1 bei einer Temperatur sowie über einen Zeitraum, die bzw. der ausreicht, um zu einer homogenen Lösung zu gelangen;
- Verursachen der Fällung eines Einschlußkomplexes durch kontrolliertes Abkühlen der so erhaltenen homogenen Lösung.

4. Verwendung nach einem der Ansprüche 1 und 2, **dadurch gekennzeichnet, daß** der genannte Komplex mit Hilfe eines Verfahrens erhalten wird, das im wesentlichen aus den folgenden Schritten besteht:
- Zugeben des Fettkörpers zu einer wäßrigen Dispersion oder Suspension des Cyclodextrins
- Erhitzen der so erhaltenen Dispersion oder Suspension auf eine Temperatur in der Größenordnung von 30 bis 70°C, vorzugsweise 40 bis 60°C, unter Rühren über einen Zeitraum zwischen 1 und 8 Stunden, vorzugsweise zwischen 1 und 3 Stunden.

5. Verwendung nach Anspruch 3 oder 4, **dadurch gekennzeichnet, daß** der genannte Einschlußkomplex zum Beispiel mittels Filtration, Trocknung oder Lyophilisation isoliert wird und dann für seine schlußendliche Verwendung in fester Form gegebenenfalls getrocknet, zerkleinert, gesiebt bzw. granuliert wird.

6. Verfahren zur Herstellung einer Emulsion, **dadurch gekennzeichnet, daß** er die Herstellung eines Cyclodextrin-Fettkörper-Einschlußkomplexes mittels Durchführung eines Verfahrens nach Anspruch 3 oder 4, dessen Dispersion in Wasser sowie die anschließende Zugabe einer Fettphase unter Rühren umfaßt.

7. Verfahren zur Herstellung einer Emulsion, **dadurch gekennzeichnet, daß** er die Herstellung eines Cyclodextrin-Fettkörper-Einschlußkomplexes mittels Durchführung eines Verfahrens nach Anspruch 3 oder 4, dessen Dispersion in einer Fettphase sowie die anschließende Zugabe von Wasser unter Rühren umfaßt.

8. Emulsionen, **dadurch gekennzeichnet, daß** sie durch die Durchführung des Verfahrens nach einem der Ansprüche 6 oder 7 erhalten werden und **dadurch**, daß sie folgendes umfassen:
- 1 bis 25 Gew.-% mindestens eines Einschlußkomplexes gemäß einem der Ansprüche 1 oder 2 oder mindestens eines Einschlußkomplexes, der durch Durchführung des Verfahrens nach einem der Ansprüche 3 oder 4 erhalten wird;
- 5 bis 75 Gew.-% Wasser; sowie
- 20 bis 90 Gew.-% einer Fettphase oder Ölphase.

## Claims

1. Use, as surfactants, of inclusion complexes of a cyclodextrin with a fatty substance, in powder form or in aqueous dispersion, **characterized in that** the molar ratio between the cyclodextrin and the fatty substance in the abovementioned complex equals 1 or less, **in that** the abovementioned cyclodextrin is selected from among β-cyclodextrin, hydroxypropyl-β-cyclodextrin or methyl-β-cyclodextrin, and **in that** the abovementioned fatty substance is a fatty alcohol selected from among the straight-chain or branched, saturated or unsaturated alcohols of natural or synthetic origin with a carbon atom number of between 8 and 36.

2. Use according to Claim 1, **characterized in that** the molar ratio between the cyclodextrin and the fatty alcohol is less than 0.75.

3. Use according to either of Claims 1 and 2, **characterized in that that** abovementioned complex is obtained by carrying out a process which consists essentially of:
- intimately mixing, in solution, at least one cyclodextrin and a fatty substance in a molar ratio of 1 or less at such a temperature and for such a period of time as to obtain a homogeneous solution;
- bringing about the precipitation of an inclusion complex by controlled cooling of the homogeneous solution thus obtained.

4. Use according to either of Claims 1 and 2, **characterized in that** the abovementioned complex is obtained by carrying out a process which consists essentially of:
- adding the fatty substance to an aqueous dispersion or suspension of cyclodextrin; and
- heating the suspension or dispersion thus obtained at a temperature in the order of 30 to 70°C, preferably 40 to 60°C, with stirring, during a period of time between 1 and 8 hours, preferably between 1 and 3 hours.

5. Use according to Claim 3 or 4, **characterized in that** the abovementioned inclusion complex is isolated, for example by filtration, drying or lyophilization, and, if appropriate, dried, comminuted, sieved, granulated for its final use in solid form.

6. Process for the preparation of an emulsion, **characterized in that** it comprises the preparation of a cyclodextrin/fatty substance inclusion complex by carrying out a process according to Claim 3 or 4, dispersing it in water and subsequently adding a fatty phase with stirring.

7. Process for the preparation of an emulsion, **characterized in that** it comprises the preparation of a cyclodextrin/fatty substance inclusion complex by carrying out a process according to Claim 3 or 4, dispersing it in a fatty phase and subsequently adding water with stirring.

8. Emulsions, **characterized in that** they are obtained by carrying out the process according to one of Claims 6 or 7 and **in that** they comprise:
- 1 to 25% by weight of at least one inclusion complex as defined in either of Claims 1 or 2 or obtained by carrying out the process according to either of Claims 3 or 4;
- 5 to 75% by weight of water; and
- 20 to 90% by weight of a fatty or oily phase.
